# EUROPEAN PATENT SPECIFICATION

(11) **EP 2 478 927 B1**
(45) Date of publication and mention of the grant of the patent: **03.01.2018**
(21) Application number: 10817051.5
(22) Date of filing: 02.09.2010
(51) Int. Cl.: A61M 25/00, A61M 25/01

(54) **CATHETER**
KATHETER
CATHÉTER

(30) Priority: 15.09.2009 JP 2009213583
(43) Date of publication of application: 25.07.2012
(73) Proprietor: Terumo Kabushiki Kaisha, Tokyo 151-0072 (JP)
(72) Inventor: TANIOKA, Hiromichi, Fujinomiya-shi Shizuoka 418-0015 (JP)
(74) Representative: Dossmann, Gérard
(86) International application number: PCT/JP2010/065020
(87) International publication number: WO 2011/033938

(56) References cited:
- WO-A1-01/70323
- WO-A1-2005/007227
- JP-A- 2000 000 309
- JP-A- 2000 042 115
- JP-A- 2001 095 923
- JP-A- 2004 130 110
- US-A1- 2003 208 221
- US-A1- 2004 087 932
- US-A1- 2004 106 866
- US-A1- 2005 197 669

## Description

### TECHNICAL FIELD

The present invention relates to a catheter which is used by being inserted inside a coelom such as a blood vessel, a vascular channel and the like.

### BACKGROUND ART

For a catheter inserted inside a coelom such as a blood vessel, a vascular channel and the like, a so-called "rapid-exchange type" catheter is used in which it is possible to carry out the pulling-in and the pulling-out of a guide wire rapidly based on a fact that there is provided, on the distal side of a catheter main body including a working lumen, with a guide wire insertion portion having a tubular shape into which the guide wire is insertable. For example, in Patent Document 1, there is described a balloon catheter of a rapid-exchange type. In this balloon catheter, there is provided a coil in the inside on the distal side of an inflation lumen (working lumen) and an anti-kink property in the vicinity of an opening portion of a guide wire insertion portion is improved by providing an extension portion which extends from that coil to the distal side away from the opening portion on the proximal side of the guide wire insertion portion. Other examples of rapid exchange catheters can be found in documents WO-A-0170323 or WO-A-2005007227.

However, with respect to the catheter described in the Patent Document 1, the coil in the inside thereof is exposed inside the working lumen. Therefore, for example, in case of applying a coil to a catheter in which a sensor is passed through inside the working lumen such as a catheter for diagnosis or the like which utilizes ultra-sound image diagnosis technology or optical coherence tomographic image diagnosis technology, there exists a possibility in which when the catheter is bent, a portion of the sensor or the like interferes with the coil and is bit into between element wires constituting the coil.

The present invention was invented in order to solve the problem mentioned above and is addressed to provide a catheter which has an excellent anti-kink property and also in which it is possible to prevent interference with respect to an object which moves inside a lumen.

### Related-Art Document

### Patent Document

Patent Document 1: Specification of European unexamined Patent publication No. 0925 801

### DISCLOSURE OF THE INVENTION

A catheter of the present invention addressed to achieve the object mentioned above is characterized by the claims and includes a catheter main body which forms a lengthy shape and also includes a lumen extending along the longitudinal direction; a guide wire insertion portion which is placed on the distal side of the catheter main body and forms a tubular shape through which a guide wire is insertable, and which includes a distal opening portion and a proximal opening portion; and a reinforcement portion provided with a coil portion in which at least a portion thereof is arranged on the proximal side away from the proximal opening portion and is wound by using an element wire, and with an extension portion formed by being expanded from the element wire of the coil portion to the distal side away from the proximal opening portion, wherein the reinforcement portion is positioned on the outside of a layer constituting an inner wall of the lumen of the catheter main body.

With respect to the catheter of the present invention, which is constituted as mentioned above, the reinforcement portion provided with the coil portion and the extension portion is positioned between the inner surface and the outer surface of the lumen of the catheter main body, so that the reinforcement portion is not exposed inside the lumen. Consequently, it is possible, while improving anti-kink property in the vicinity of the proximal opening portion by the reinforcement portion, to prevent the object moving inside the lumen from interfering with the reinforcement portion.

In addition, if it is constituted such that the extension distal portion which is the end portion on the distal side of the extension portion is formed by a curved portion which is formed by bending the element wire, the extension distal portion does not exert damage to the material constituting the catheter and is not exposed inside the lumen or inside the coelom, so that the safety thereof is excellent.

If it is constituted with respect to the reinforcement portion such that a bending portion formed by bending one line of element wire is made to be the extension distal portion, and the coil portion is formed by winding the opposite side of the bending portion of the element wire in a coil shape, a catheter having high safety can be manufactured easily and the work performance thereof when manufacturing is excellent.

If it is constituted with respect to the extension portion such that the bending rigidity thereof decreases from the proximal side toward the distal side in an inclined fashion or in a stepwise fashion, lower rigidity and higher flexibility toward the distal side can be realized in which it is possible to change the bending rigidity of the catheter smoothly and it is possible to improve anti-kink property.

If it is constituted such that the element wire constituting the extension portion is diameter-thinned toward the distal side thereof, it is possible to easily realize a constitution having lower rigidity and higher flexibility toward the distal side.

If it is constituted such that the extension portion is positioned between the center axis of the guide wire insertion portion and the center axis of the catheter main body, it is possible for the extension portion to be arranged while being contacted with both the guide wire insertion portion and the catheter main body and it is possible for the bending rigidity between the guide wire insertion portion and the catheter main body to be reinforced efficiently. In addition, it is possible for the extension portion to be arranged approximately in parallel with both the center axis of the guide wire insertion portion and the center axis of the catheter main body, so that it is possible to repress deviation of the bending rigidity which is caused by inclination with respect to the center axis.

If it is constituted such that the reinforcement portion is composed of a metal material, it is possible to secure the reinforcement strength depending on the reinforcement portion sufficiently.

### BRIEF DESCRIPTION OF DRAWINGS

FIG. 1 is a plan view showing a catheter relating to a first exemplified embodiment;
FIG. 2 is a cross-sectional view in a longitudinal direction showing a distal side of a catheter relating to the first exemplified embodiment;
FIG. 3 is a cross-sectional view in a longitudinal direction showing a distal side of a catheter relating to the first exemplified embodiment;
FIG. 4 is a cross-sectional view in a longitudinal direction showing a proximal side of a guide wire insertion portion of a catheter relating to the first exemplified embodiment;
FIG. 5 is a plan view showing a reinforcement coil of a catheter relating to the first exemplified embodiment;
FIG. 6 is a cross-sectional view in a longitudinal direction showing a proximal side of a guide wire insertion portion when a catheter relating to the first exemplified embodiment is bent;
FIG. 7 is a plan view showing a reinforcement coil used for a catheter relating to a second exemplified embodiment;
FIG. 8 is a plan view showing a reinforcement coil used for a catheter relating to a third exemplified embodiment;
FIG. 9 is a plan view showing a reinforcement coil used for a catheter relating to a fourth exemplified embodiment;
FIG. 10 is a cross-sectional view in a longitudinal direction showing a proximal side of a guide wire insertion portion of a catheter relating to a fifth exemplified embodiment; and
FIG. 11 is a cross-sectional view in a longitudinal direction showing a proximal side of a guide wire insertion portion of a catheter relating to a sixth exemplified embodiment.

### BEST MODE FOR CARRYING OUT THE INVENTION

### <First Exemplified Embodiment>

Hereinafter, exemplified embodiments relating to the present invention will be explained with reference to the drawings. It should be noted that there exists a case in which the size ratio of the drawing is different from the actual ratio by being exaggerated for convenience of explanation.

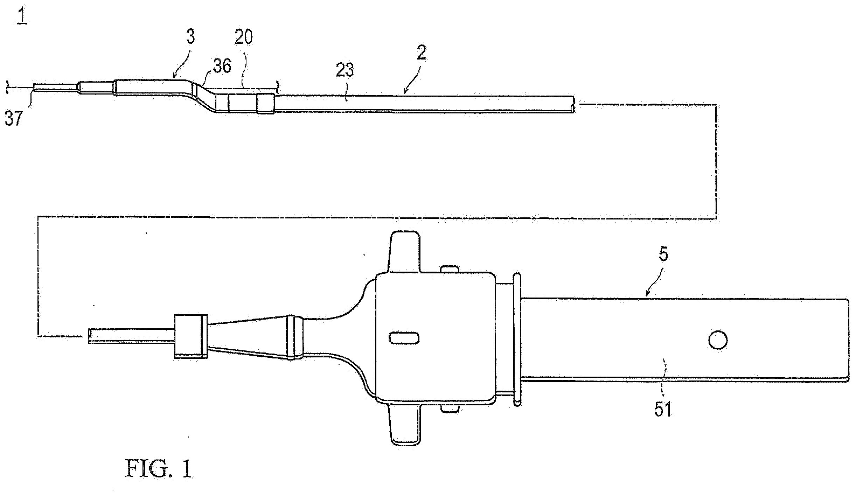

A catheter 1 relating to a first exemplified embodiment of the present invention is, as shown in FIG. 1, provided with a catheter main body 2 having a lengthy shape and also flexibility, which is inserted inside a living body such as a coelom and the like; a guide wire insertion portion 3 installed on the distal side of the catheter main body 2 and through which a guide wire 20 is insertable; and a connector 5 which is installed on the proximal side of the catheter main body 2 and which is arranged on the operator's hand side without being inserted inside the coelom in order to be steered by the operator. The catheter 1 is a so-called "rapid-exchange type" catheter 1 by which it is possible to carry out the pulling in and out of the guide wire 20 rapidly. It should be noted in this specification that the side from which the catheter 1 is inserted inside the coelom is referred to as distal side and the side not inserted inside the coelom therethrough is referred to as proximal side.

The guide wire 20 is inserted beforehand until the vicinity of the target lesion inside the living body before the catheter 1 is inserted inside the living body and used for introducing the catheter 1 until the target lesion. The catheter 1 is introduced until the target lesion while passing the guide wire 20 through a guide wire lumen 32 formed at the above-mentioned guide wire insertion portion 3.

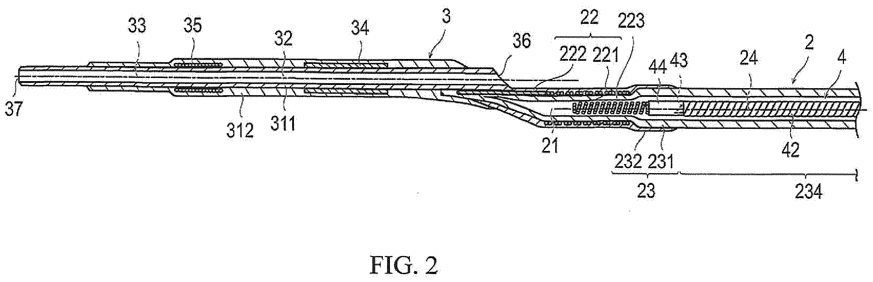

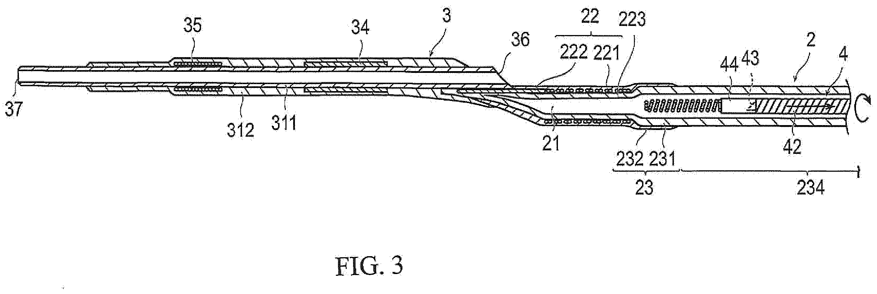

The catheter main body 2 is provided, as shown in FIG. 2 and FIG. 3, with a working lumen 21 (Lumen) which extends along the longitudinal direction. This working lumen 21 is a hollow passage in which an image obtaining unit 4 mentioned later moves.

A tube wall 23 which defines the working lumen 21 of the catheter main body 2 is constituted by a first layer 231 inside which the working lumen 21 is formed and a second layer 232 covering the outer circumferential surface on the distal side of the first layer 231. For the material of the first layer 231, there is no limitation in particular, but there can be cited, for example, such as: fluorine-based resin such as PFA (tetrafluoroethylene-perfluoroalkylvinylether copolymer), FEP (tetrafluoroethylene-hexafluoropropylene copolymer), ETFE (tetrafluoroethylene-ethylene copolymer) and PTFE (polytetrafluoroethylene); pollyamide; polyimide; polyolefin such as polyethylene and polypropylene; polyester such as polyethylene terephthalate and polybutylene terephthalate; polyurethane polystyrene; polycarbonate; silicon resin; and polyetherimide, and within those above, it is preferable to employ a material such as high density polyethylene which has low friction property and also which has high signal permeability. Thus, the friction resistance of the inner surface of the working lumen 21 becomes small and it is possible to move the drive shaft 42 smoothly inside the working lumen 21. It is allowed for the first layer to be formed by a plurality of layers composed of the materials selected from the above-mentioned materials. For the material of the second layer 232, there is no limitation in particular, but there can be cited, for example, such as: various kinds of thermoplastic elastomers such as polyurethane elastomer, polyester elastomer and polyamide elastomer; pollyamide; polyimide; polyolefin such as polyethylene and polypropylene; polyurethane; silicon resin; polyester such as polyethylene terephthalate and polybutylene terephthalate; or a mixture made by mixing two or more kinds of those above, and within those above, it is preferable to employ materials such as various kinds of thermoplastic elastomers, polyurethane and silicon resin which are rich in flexibility comparatively. Thus, it is possible to prevent a phenomenon in which a coelom inside of a living body into which the catheter 1 is inserted is to be damaged.

It is constituted for the tube wall 23 of the catheter main body 2 such that on the proximal side from the portion of the first layer 231 which is covered by the second layer 232, there is provided a window portion 234 passing through a signal such as a light, an ultra-sound and the like. The catheter 1 relating to this exemplified embodiment is a catheter 1 which obtains an image by an optical signal utilizing an optical coherence tomographic image diagnosis technology (Optical Coherent Tomography: OCT) and it is possible to obtain a tomographic image by transmitting and receiving light through the window portion 234.

With respect to the second layer 232, the distal side portion thereof extends to a region on the proximal side of the guide wire insertion portion 3 and it covers the circumference of a proximal opening portion 36 of the guide wire lumen 32. Thus, the vicinity of the proximal opening portion 36 of the guide wire insertion portion 3 is reinforced and occurrence of kink can be repressed.

The guide wire insertion portion 3 is provided with an inner layer 311 and an outer layer 312 which form tubular shapes. The guide wire insertion portion 3 includes a distal opening portion 37 at which the guide wire lumen 32 opens on the distal side and the afore-mentioned proximal opening portion 36 which opens on the proximal side. With respect to the proximal portion of the guide wire insertion portion 3, a portion thereof is interlinked overlappingly with the distal portion of the catheter main body 2. Thus, while maintaining high interlinking strength, there is avoided a large-diameter tendency of the distal portion of the catheter 1. The guide wire insertion portion 3 and the catheter main body 2 are fixed by thermal bonding in this exemplified embodiment, but the fixing method is not limited by this aspect.

For the guide wire insertion portion 3, the center axis 33 thereof is arranged eccentrically with respect to the center axis 24 of the catheter main body 2. Thus, in the vicinity of the fixing region of the guide wire insertion portion 3 with respect to the catheter main body 2, the guide wire lumen 32 can be secured broadly and also in an upright state, so that it is possible to pass through the guide wire 20 more smoothly.

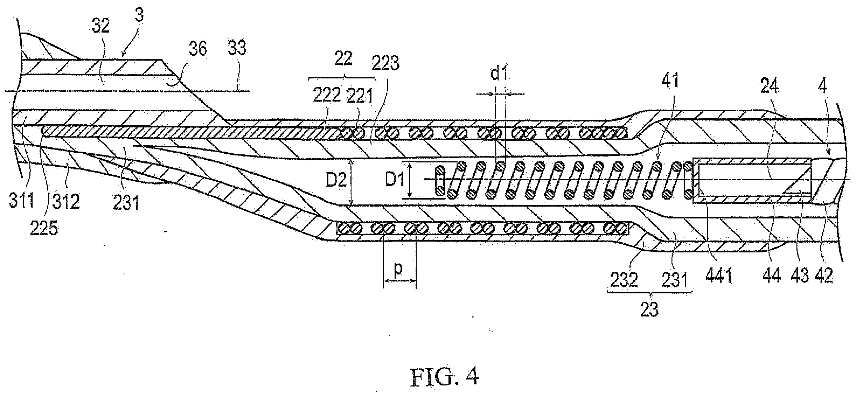

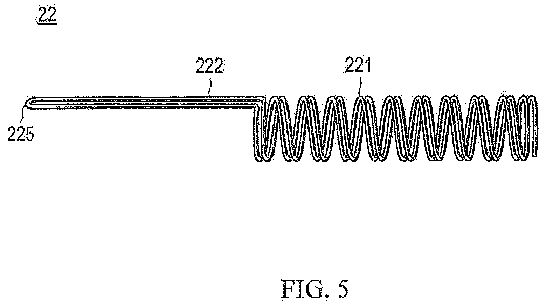

In the vicinity of the boundary of the catheter main body 2 and guide wire insertion portion 3, there is buried and placed a reinforcement coil 22 (reinforcement portion) for reinforcing bending rigidity between the catheter main body 2 and the guide wire insertion portion 3 and for repressing kink. The reinforcement coil 22 includes, as shown in FIG. 4 and FIG. 5, a coil portion 221 formed by winding an element wire in a spiral shape and an extension portion 222 projecting by a configuration that an element wire is expanded from one end side of the coil portion 221.

The coil portion 221 is buried and placed concentrically with the working lumen 21 at the distal portion of the working lumen 21 so as to be sandwiched between the first layer 231 and the second layer 232. Thus, in the catheter main body 2, the exposure of the coil portion 221 to the working lumen 21 is blocked and the region in which this exposure is blocked is referred to as an exposure stopper portion 223. With respect to the exposure stopper portion 223, the inner diameter and the outer diameter thereof are diameter-reduced in which the amount of diameter-reduction of the inner diameter is smaller than the amount of diameter-reduction of the outer diameter. Therefore, it becomes in a constitution such that the wall thickness of the catheter main body 2 on the side of the exposure stopper portion 223 is smaller than that on the proximal side of the tube wall 23. Also, on the exposure stopper portion 223, the second layer 232 is coated. In this manner, while attempting diameter-thinning at the distal portion of the catheter 1, a reasonable rigidity is maintained by providing the coil portion 221 and by coating the second layer 232.

The extension portion 222 extends from the distal side of the coil portion 221 to the distal side away from the proximal opening portion 36 of the guide wire lumen 32, and it is buried and placed between the first layer 231 of the catheter main body 2 and the inner layer 311 of the guide wire insertion portion 3. In this exemplified embodiment, depending on a fact that the surface on the proximal side of the guide wire insertion portion 3 is formed by being inclined, the proximal opening portion 36 is opened by possessing a certain area along the longitudinal direction of the guide wire insertion portion 3 and the extension portion 222 of the reinforcement coil 22 extends until the distal side away from the whole of the area over which the proximal opening portion 36 spreads. It should be noted that it is allowed even if the extension portion 222 of the reinforcement coil 22 does not always extend until the distal side away from the whole of the area over which the proximal opening portion 36 spreads and it is also allowed to be formed so as to extend until the distal side away from at least the region on the most proximal side of the proximal opening portion 36.

Also, it is allowed for the extension portion 222 to be extended until the distal side away from the first layer 231 of the catheter main body 2 and to be buried and placed between the inner layer 311 and the outer layer 312 of the guide wire insertion portion 3.

It is preferable for the extension portion 222 to be positioned between the center axis 33 of the guide wire insertion portion 3 and the center axis 24 of the catheter main body 2. Thus, it is possible to arrange the extension portion 222 while being contacted with both the guide wire insertion portion 3 and the catheter main body 2, and it is possible to reinforce the bending rigidity between the guide wire insertion portion 3 and the catheter main body 2 efficiently. Also, it is possible to arrange the extension portion 222 approximately in parallel with both the center axis 33 of the guide wire insertion portion 3 and the center axis 24 of the catheter main body 2, so that it is possible to repress the deviation of the bending rigidity, which is caused by the inclination with respect to the center axis.

With respect to the reinforcement coil 22, one line of element wire is made to be doubled by being bent in a U-shape and thereafter, an end portion of the bending portion bent in a U-shape is made to be an extension distal portion 225 of the extension portion 222, and the coil portion 221 is to be formed by winding the side on which the end portions of the element wire are overlapped each other. Consequently, the extension distal portion 225 of the extension portion 222 is formed by a continuous wire of an element wire which is folded back, so that it is formed smoothly. For this reason, for example, even in a case in which the vicinity of the proximal opening portion 36 is deformed significantly, the extension distal portion 225 does not exert damage on the material constituting the catheter 1 and is not exposed inside the guide wire lumen 32, inside the working lumen 21 or inside the coelom, so that there is realized a catheter 1 which is excellent in safety. Also, by any possibility, even if the extension distal portion 225 is exposed inside the guide wire lumen 32, inside the working lumen 21 or inside the coelom, the extension distal portion 225 is formed smoothly, so that the safety thereof can be secured. Also, only by winding an element wire after being bent, it is easily possible to realize a safely constitution and the work performance on an occasion of the manufacture is excellent.

In this exemplified embodiment, the coil portion 221 is formed by winding a doubled element wire with a constant pitch p, but the pitch p is changeable variously and, for example, it is also possible to wind the coil by a close coiling such that adjoining element wires contact with each other.

It should be noted that it is allowed to employ a constitution with respect to the element wire constituting the reinforcement coil 22 such that by using a ring shaped element wire, the element wire is folded back so as to be continuous at both the end portions which became doubled. Also, it is allowed even if the constitution is accomplished not always by a doubled element wire and it is allowed to employ a single wire, or a tripled or more multiple-folded wire.

The element wire is produced by such as: a stainless steel; a spring steel; a super elastic alloy; a cobalt-based alloy; and a noble metal such as gold, platinum, platinum/iridium alloy and tungsten, or by such as: an alloy containing those metals above, and within them, it is preferable to employ a super elastic alloy, in particular, a Ni-Tisuper elastic alloy. If forming the element wire by a metallic material, it is possible to secure the reinforcement strength by the reinforcement coil 22 sufficiently.

As one example, in this exemplified embodiment, the element wire is produced by a Ni-Tisuper elastic alloy having a wire-diameter of 0.05mm, the inner diameter D1 of the coil portion 221 is 0.64mm, the outer diameter D2 thereof is 0.74mm, the pitch P thereof is 0.2mm, and the length L of the extension portion 222 is 2mm.

For the guide wire insertion portion 3, as shown in FIG. 2 and FIG. 3, there are arranged a reinforcement tube 34 and a marker 35 between the inner layer 311 and the outer layer 312.

The marker 35 is a maker which makes the distal end position of the catheter 1 confirmable under X-ray fluoroscopy when being inserted inside the living body, and it is formed by winding an element wire in a spiral shape and it is wound in close coiling such that the neighboring elements thereof are wound so as to be contacted with each other.

It is preferable for the element wire to be a metal material having X-ray impermeability such as: a stainless steel, a super elastic alloy, a cobalt-based alloy, gold, platinum, a platinum/iridium alloy, tungsten and tantalum, and it is produced by a noble metal, an alloy containing those metals or the like. It is allowed for the marker 35 to be in a pipe shape.

It should be noted that the marker 35 is a maker which has contrast imaging property under X-ray fluoroscopy caused by a reason that it has an X-ray non-permeability, and usually, such a marker 35 has contrast imaging property also for CT scanning, so that it can be used also in the CT scanning.

The reinforcement tube 34 is a tubular member which is arranged at the proximal portion of the guide wire insertion portion 3.

In a case in which the guide wire 20 which passes through the guide wire lumen 32 and is exposed from the proximal opening portion 36 toward the proximal side is curved and is apart from the catheter main body 2 toward the side thereof during the steering of the catheter 1, there exists a fear that the proximal side apart from the reinforcement tube 34 of the guide wire insertion portion 3 is to be split caused by the guide wire 20, but the reinforcement tube 34 regulates deformation of the guide wire 20 and fulfills a function of repressing easily occurring split of the guide wire insertion portion 3.

It is allowed for the reinforcement tube 34 to be formed with a spiral shaped groove (not shown) which is wound around the center axis. The groove can be formed on at least one side of the inner circumferential surface and the outer circumferential surface or it can be formed also on both the sides thereof so as to be opened. By providing such a groove, it is possible to keep flexibility of the guide wire insertion portion 3 at which the reinforcement tube 34 is installed and even if inserting the catheter 1 inside a bent lumen of a living body, following-ability of the guide wire insertion portion with respect to the lumen of the living body is exerted reliably.

Consequently, in the guide wire insertion portion 3, by providing the reinforcement tube 34 having a groove, it is possible to obtain both the conflicting effects of "reinforcement" and "securement of flexibility" at the same time.

For the resin material constituting the reinforcement tube 34, there is no limitation in particular and, for example, it can be cited such as: PTFE (polytetrafluoroethylene); FEP (tetrafluoroethylene-hexafluoropropylene copolymer); PFA (tetrafluoroethylene-perfluoroalkylvinylether copolymer); ETFE (tetrafluoroethylene-ethylene copolymer); polyolefin such as polyethylene, polypropylene and ethylene-vinyl acetate copolymer; modified polyolefin; pollyamide (example: nylon 6; nylon 46; nylon 66; nylon 610; nylon 612; nylon 11; nylon 12; nylon 6-12; nylon 6-66); thermoplastic polyimide; thermosetting polyimide; liquid crystal polymer such as aromatic polyester; polyphenylene oxide; polyphenylene sulfide; polycarbonate; polymethyl methacrylate; polyether; polyetherketone; polyetherimide; polyacetal; various kinds of thermoplastic elastomers such as stylene-based elastomer, polyolefin-based elastomer, polyvinyl chloride-based elastomer, polyurethane-based elastomer, polyester-based elastomer, pollyamide-based elastomer, polybutadiene-based elastomer, trans-polyisoprene-based elastomer, fluorine rubber-based elastomer and chlorinated polyethylene-based elastomer; or a copolymer, a blended body and a polymer alloy, which make those above to be main materials, and it is possible to use one kind or to use two kinds or more by mixing materials within those above.

The image obtaining unit 4 which is arranged inside the working lumen 21 of the catheter 1 is, as shown in FIGS. 1 to 3, provided with a prism 43 for transmitting and receiving the light toward the tissue inside the coelom, a housing 44 for housing the prism 43, a drive shaft 42 for mounting the housing 44 on the distal end thereof and concurrently for transmitting a rotational motive force, a rotation stabilizing coil 41 attached to the distal side of the housing 44, and a scanner 51. The winding direction of the rotation stabilizing coil 41 is same as the winding direction of the reinforcement coil 22, but it is allowed to employ opposite direction.

The prism 43 is a right-angle prism and as shown in FIG. 4, it is fixed at the distal end (distal end of optical fiber) of the drive shaft 42 which is movable forward and backward inside the working lumen 21. For the fixing method thereof, there is no limitation in particular and it is possible to accomplish the bonding, for example, by an adhesive agent or by soldering.

The housing 44 is formed in a tubular shape having a distal end closing portion 441 in which the distal side thereof is closed, and the proximal side thereof is fixed on the drive shaft 42. For the fixing method thereof, there is no limitation in particular and it is possible to accomplish the bonding, for example, by an adhesive agent or by soldering. With respect to the housing 44, an opening portion thereof is formed by cutting off a portion corresponding to the transmitting & receiving portion of the light of the prism 43.

The rotation stabilizing coil 41 is formed by winding an element wire in a spiral shape. For the rotation stabilizing coil 41, the proximal side thereof is fixed at the distal end closing portion 441 of the housing 44 and it becomes a guide for rotating the prism 43 stably. For the fixing method thereof, there is no limitation in particular similarly as mentioned before and it is possible to accomplish the bonding, for example, by an adhesive agent or by soldering.

It is preferable for the rotation stabilizing coil 41 to be produced by a metal material and for example, it is produced by a metal material having X-ray impermeability such as: a spring steel, a stainless steel, a super elastic alloy, a cobalt-based alloy, gold, platinum and tungsten, or by such as an alloy containing those metals. It should be noted that it is also possible for the rotation stabilizing coil 41 to be manufactured by a material other than the metal material.

The rotation stabilizing coil 41 is housed inside the exposure stopper portion 223 when the drive shaft 42 is pressed toward the distal side maximally. At that time, the movement of the drive shaft 42 is regulated by a limit switch of an axis direction move stage (not shown) of the scanner and the pressing is stopped, so that it is constituted such that the drive shaft is pressed onto the exposure stopper portion 223 and is not to be destructed.

When the rotation stabilizing coil 41 is housed in the exposure stopper portion 223, it happens that the reinforcement coil 22 and the rotation stabilizing coil 41 are overlapped, but the reinforcement coil 22 is covered by the first layer 231 and is not exposed to the inside of the working lumen 21, so that it is possible to reliably prevent the biting between the coils each other of the reinforcement coil 22 and the rotation stabilizing coil 41.

Also, when the rotation stabilizing coil 41 is entered into the exposure stopper portion 223, it becomes in a state in which the rotation stabilizing coil 41 is overlapped on the inside of the reinforcement coil 22, so that at the distal end of the catheter main body 2, the image obtaining unit 4 and the catheter main body 2 become in one-body configuration and when inserting the catheter 1 inside the living body, there can be realized a structure which has a strong resistance against the bending thereof (anti-kink structure).

The drive shaft 42 has a characteristic of being flexible and also, in which it is possible for a rotational motion power produced in the connector 5 to be transmitted to the prism 43 and, for example, it is constituted by a tube body with a multilayer coil shape to have a constant outer diameter.

There is connected a connector 5 on the proximal side of the catheter main body 2. The connector 5 is provided, for example, with an optical fiber connector in the inside thereof, and it is possible to connect the drive shaft 42 having an optical fiber in the inside thereof to a scanner 51 for rotating the aforesaid drive shaft 42 in a high speed. It is possible for the scanner 51 to communicate the optical signal from the prism 43, which is transmitted through the optical fiber inside the drive shaft 42, to an exclusive analyzing apparatus. In this communication state, it is possible to display the tomographic image inside the lumen of the living body, which is constructed by the analyzing apparatus, on an image display apparatus.

When obtaining an image by the image obtaining unit 4, as shown in FIG. 2 and FIG. 3, the drive shaft 42 is pressed toward the distal side maximally and while moving the drive shaft 42 toward the proximal side along the working lumen 21 from an insertion state in which the rotation stabilizing coil 41 is inserted into the reinforcement coil 22, the drive shaft 42 is steered so as to rotate around the axis thereof. In synchronized with the spiral motion of such a drive shaft 42, also the prism 43 moves in spiral and it is possible to obtain a tomographic image over the longitudinal direction inside the coelom.

When obtaining a tomographic image again, the drive shaft 42 is moved toward the distal side and by setting a state as shown in FIG. 2, it is possible to implement an image obtaining process again. At that time, there is provided with the exposure stopper portion 223, so that it is possible for the operator to insert the rotation stabilizing coil 41 into the reinforcement coil 22 without any contact and it is possible to prevent a damage and a biting of the reinforcement coil 22 and the rotation stabilizing coil 41. Further, the exposure stopper portion 223 functions also as a push-in stopper for stopping the movement of the drive shaft 42, so that it is possible to arrange the drive shaft 42 at an appropriate position reliably.

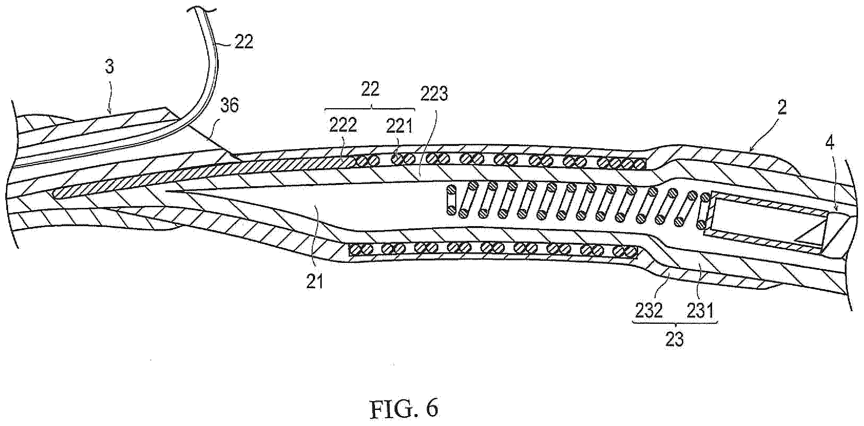

As mentioned above, it is possible for the catheter 1 to take an insertion state in which the rotation stabilizing coil 41 is inserted into the reinforcement coil 22 and in this state, for example, in a case in which a pressing force is acted on the guide wire 20 and this pressing force is transmitted to the guide wire insertion portion 3, as shown in FIG. 6, a bending occurs in a vicinity of the boundary portion between the catheter main body 2 and the guide wire insertion portion 3. Even if such a bending occurs, the exposure stopper portion 223 intervenes between the rotation stabilizing coil 41 and the reinforcement coil 22, so that there can be prevented "bating" in which both the element wires interleave each other and there is no interference therebetween. Consequently, if the afore-mentioned pressing force is canceled, as shown in FIG. 2 and FIG. 4, the rotation stabilizing coil 41 and the reinforcement coil 22 can restore the original shapes, and there is realized a catheter 1 which has an excellent anti-kink property.

Also, in an insertion state shown in FIG. 4 and in a natural state in which a bending does not occur, the outer circumferential portion of the rotation stabilizing coil 41 is apart from the exposure stopper portion 223. It should be noted that it is preferable for the difference between the maximum outer diameter D1 of the rotation stabilizing coil 41 and the inner diameter D2 of the exposure stopper portion 223 to be selected identical or less than the wire-diameter dl of the element wire of the rotation stabilizing coil 41. In a case in which the rotation stabilizing coil 41 is apart from the exposure stopper portion 223, even if the bending occurs at the reinforcement coil 22, it is possible to prevent the exposure stopper portion 223 from contacting with the rotation stabilizing coil 41 for a case depending on the degree of bending. Even if the exposure stopper portion 223 supposedly contacts to the rotation stabilizing coil 41, the influence exerted to the rotation of the drive shaft 42 is very small.

Also, with respect to the reinforcement coil 22, the extension portion 222 thereof extending from the coil portion 221 is extended until the distal side away from the proximal opening portion 36 of the guide wire insertion portion 3, so that it is possible to improve the bending rigidity in the vicinity of the proximal opening portion 36 of the distal side away from the coil portion 221. Thus, there is improved the anti-kink property in the vicinity of the proximal opening portion 36 in which the rigidity becomes discontinuous and a kink occurs easily. In particular, the extension portion 222 is extended from the element wire of the coil portion 221 and is formed continuously, so that also the bending rigidity becomes continuous and there is obtained an excellent anti-kink property.

Also, the reinforcement coil 22 is provided within narrow range of the boundary portion between the guide wire insertion portion 3 and the catheter main body 2, and it is possible to heighten the rigidity thereof, so that it is possible to arrange the prism 43 on the distal side as much as possible inside the catheter 1. Consequently, when inserting the catheter 1 inside the coelom, the tomographic image can be obtained from the distal side as much as possible of the catheter 1, so that it is possible to widen the measurement range or it is possible to reduce the burden on a patient by reducing the insertion amount of the catheter 1 inside the coelom.

Also, it is allowed for the reinforcement coil 22 to be buried and placed on the respective insides of the first layer 231 and the second layer 232.

### <Second Exemplified Embodiment>

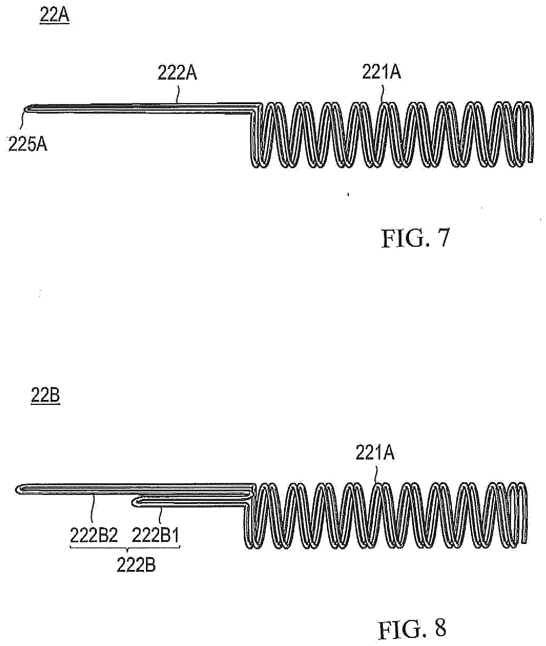

Hereinafter, with reference to FIG. 7, there will be explained a catheter relating to a second exemplified embodiment of the present invention centering around different aspects with respect to the first exemplified embodiment and for the similar matters, the explanation thereof will be omitted.

The catheter relating to the second exemplified embodiment of the present invention includes, as shown in FIG. 7, a reinforcement coil 22A which is provided with a coil portion 221A and an extension portion 222A extending from the coil portion 221A, and the element wire constituting the extension portion 222A is formed so as to become finer in an inclined fashion from the proximal side of the extension portion 222A toward an extension distal portion 225A. It should be noted that the position at which the reinforcement coil 22A is placed in the catheter is similar to that of the first exemplified embodiment. If employing such a constitution, the extension portion 222A has lower rigidity and more flexibility toward the distal side and it is possible to change the bending rigidity smoothly from the region which is provided with the reinforcement coil 22A in the catheter toward the guide wire insertion portion 3, and it is possible to improve the anti-kink property of the catheter.

For a method of narrowing the extension portion 222A toward the distal side, there can be cited, for example, a chemical polishing method. In a case in which the reinforcement coil 22A is made of a Ni-Tisuper elastic alloy, by using a chemical of Hydrofluoric acid or the like and by adjusting an immersion time with respect to the chemical, it is possible to make the wire-diameter of the element wire finer in an inclined fashion.

### <Third Exemplified Embodiment>

Hereinafter, with reference to FIG. 8, there will be explained a catheter relating to a third exemplified embodiment of the present invention centering around different aspects with respect to the first exemplified embodiment and for the similar matters, the explanation thereof will be omitted.

The catheter relating to the third exemplified embodiment of the present invention includes, as shown in FIG. 8, a reinforcement coil 22B in which a plurality of extension portions 222B (222B1, 222B2) are formed by folding back the element wire extending from a coil portion 221B by a plurality of times. Then, by selecting the lengths of the plurality of extension portions 222B1, 222B2 to be different, it is possible to change the bending rigidity in a stepwise fashion. More specifically, in this exemplified embodiment, by providing the short extension portion 222B1 and the long extension portion 222B2, the number of the element wires contributing to the bending rigidity is reduced from four pieces to two pieces from the proximal side toward the distal side, so that it is constituted such that the bending rigidity of the distal side in the extension portion 222B is smaller than that of the proximal side. It should be noted that the position at which the reinforcement coil 22B is placed in the catheter is similar to that of the first exemplified embodiment. If employing such a constitution, it is possible to reduce the bending rigidity of the reinforcement coil 22B in a stepwise fashion from the proximal side toward the distal side, so that it is possible to change the bending rigidity in the catheter smoothly and it is possible to improve the anti-kink property of the catheter. It should be noted that the number of the extension portions 222B is not limited by two pieces and it is allowed to employ three pieces or more. Also, with respect to the respective lengths of the plurality of extension portions 222B1, 222B2, there is not only a case in which those are different but also there is a case in which those are made equal.

### <Fourth Exemplified Embodiment>

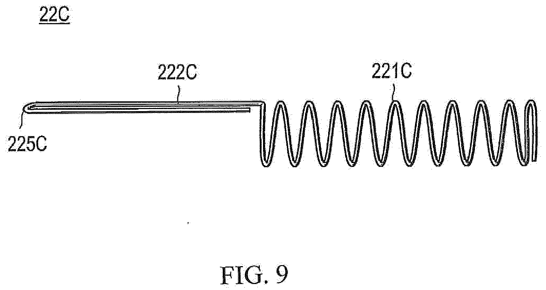

Hereinafter, with reference to FIG. 9, there will be explained a catheter relating to a fourth exemplified embodiment of the present invention centering around different aspects with respect to the first exemplified embodiment and for the similar matters, the explanation thereof will be omitted.

The catheter relating to the fourth exemplified embodiment of the present invention includes, as shown in FIG. 9, a reinforcement coil 22C in which a coil portion 221C is constituted by a single-phase wire coil and an extension portion 222C is constituted by folding back the element wire extending from the coil portion 221C at an extension distal portion 225C. It should be noted that the position at which the reinforcement coil 22C is placed in the catheter is similar to that of the first exemplified embodiment. Depending also on such a constitution, similarly as the first exemplified embodiment, it is possible to form the extension distal portion 225C of the reinforcement coil 22C in a smooth shape and it is possible to secure the safety.

### <Fifth Exemplified Embodiment>

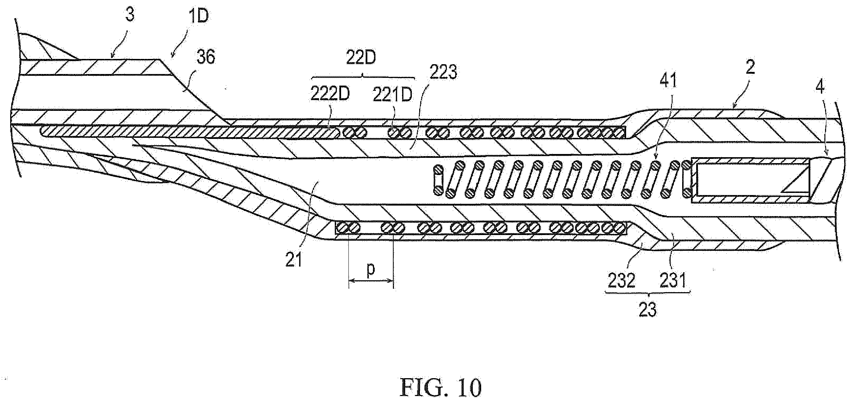

Hereinafter, with reference to FIG. 10, there will be explained a catheter 1D relating to a fifth exemplified embodiment of the present invention centering around different aspects with respect to the first exemplified embodiment and for the similar matters, the explanation thereof will be omitted.

With respect to the catheter 1D relating to the fifth exemplified embodiment of the present invention, as shown in FIG. 10, pitch p thereof between adjacent element wires of a coil portion 221D of a reinforcement coil 22D which includes the coil portion 221D and an extension portion 222D is different along the longitudinal direction. The pitch p is set to be narrower toward the proximal side and to be broader toward the distal side. If employing such a constitution, the bending rigidity of the reinforcement coil 22D decreases in an inclined fashion from the proximal side toward the distal side, so that it is possible to change the bending rigidity for the catheter 1D smoothly and it is possible to improve anti-kink property in the vicinity of the proximal opening portion 36 of the catheter 1D.

### <Sixth Exemplified Embodiment>

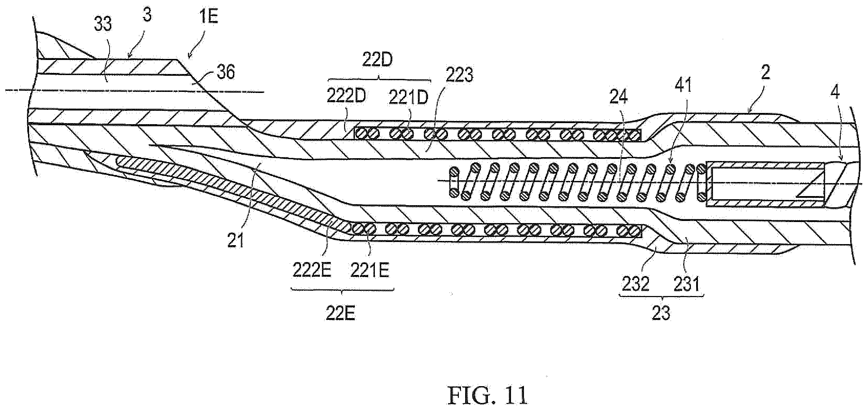

Hereinafter, with reference to FIG. 11, there will be explained a catheter 1E relating to a sixth exemplified embodiment of the present invention centering around different aspects with respect to the first exemplified embodiment and for the similar matters, the explanation thereof will be omitted.

With respect to the catheter 1E relating to the sixth exemplified embodiment of the present invention, as shown in FIG. 11, an extension portion 222E thereof extending from a coil portion 221E of a reinforcement coil 22E is not positioned between the center axis 33 of the guide wire insertion portion 3 and the center axis 24 of the catheter main body 2, but is positioned on the opposite side of the center axis 33 of the guide wire insertion portion 3 with respect to the center axis 24 of the catheter main body. In this manner, it is also possible to set the circumferential directional position at which the extension portion 222E of the reinforcement coil 22E is arranged to be at a different position from the that of the first exemplified embodiment in which it was provided between the center axis 33 and the center axis 24. Even if employing such a constitution, it is possible to change the bending rigidity in the vicinity of the proximal opening portion 36 of the catheter 1E smoothly and it is possible to improve anti-kink property of the catheter 1E. It should be noted that with respect to the circumferential direction position at which the extension portion 222E is expanded, it is not limited by those aspects and the position can be changed appropriately.

It should be noted that the present invention is not limited by the exemplified embodiments mentioned above and it is possible to make alteration variously within the scope of claims. For example, in the exemplified embodiments mentioned above, it was explained with respect to a case in which the present invention is applied to a catheter for diagnosis utilizing an optical coherence tomographic image technology (OCT), but it is also possible to apply the present invention to another catheter for diagnosis. For example, it is possible to apply the present invention to an ultra-sound catheter utilizing an ultrasonic transducer. In the ultra-sound catheter, it is possible to observe a living body by entering an ultra-sound into the living body and based on the ultra-sound returning after being scattered or being absorbed, or after being reflected or being refracted inside the living body. Consequently, it is possible for the detection wave to apply not only the light but also all kinds of waves which are applicable for the detection of such as ultra-sound, magnetic field, sound and the like. In addition, it is also possible to apply a plurality of features of the exemplified embodiments mentioned above in combination.

Further, this patent application is based on Japanese Patent Application No.2009-213583, filed September 15, 2009.

### DESCRIPTION OF REFERENCE NUMERALS

1, 1D, 1E: catheter;
2: catheter main body;
3: guide wire insertion portion;
20: guide wire;
21: working lumen (lumen) ;
22, 22A, 22B, 22C, 22D, 22E: reinforcement coil (reinforcement portion);
24: center axis of catheter main body;
32: guide wire lumen;
33: center axis of guide wire insertion portion;
36: proximal opening portion;
37: distal opening portion;
221, 221A, 221B, 221C, 221D, 221E: coil portion;
222, 222A, 222B1, 222B2, 222C, 222D, 222E: extension portion;
223: exposure stopper portion;
225, 225A, 225C: extension distal portion;
231: first layer;
232: second layer.

## Claims

1. A catheter (1, 1D, 1E) **characterized by** comprising:
a catheter main body (2) which forms a lengthy shape and also includes a lumen (21) extending along the longitudinal direction;
a guide wire insertion portion (3) which is placed on the distal side of the catheter main body (2) and forms a tubular shape through which a guide wire is insertable, and which includes a distal opening portion (37) and a proximal opening portion (36); and
a reinforcement portion (22, 22A, 22B, 22C, 22D, 22E) provided with a coil portion (221, 221A, 221B, 221C, 221D, 221E) in which at least a portion thereof is arranged on the proximal side away from the proximal opening portion (36) and is wound by using an element wire, **characterised in that** said reinforcing portion further comprises an extension portion (222, 222A, 222B 1, 222B2, 222C, 222D, 222E) formed by being expanded from the element wire of the coil portion (221, 221A, 221B, 221C, 221D, 221E) to the distal side away from the proximal opening portion (36), wherein the reinforcement portion (22, 22A, 22B, 22C, 22D, 22E) is positioned between the inner surface and the outer surface of a tube wall (23) defining the lumen (21) of the catheter main body (2).

2. The catheter (1, 1D, 1E) according to claim 1, **characterized in that** the extension distal portion (225, 225A, 225C) which is the end portion on the distal side of the extension portion (222, 222A, 222B1, 222B2, 222C, 222D, 222E)is formed by a curved portion which is formed by bending the element wire.

3. The catheter (1, 1D, 1E) according to claim 2, **characterized in that** with respect to the reinforcement portion (22, 22A, 22B, 22C, 22D, 22E), a bending portion formed by bending one line of element wire is made to be the extension distal portion (225, 225A, 225C), and the coil portion (221, 221A, 221B, 221C, 221D, 221E) is formed by winding the opposite side of the bending portion of the element wire in a coil shape.

4. The catheter (1, 1D, 1E) according to any one of claims 1 to 3, **characterized in that** with respect to the extension portion (222, 222A, 222B1, 222B2, 222C, 222D, 222E), the bending rigidity thereof decreases from the proximal side toward the distal side in an inclined fashion or in a stepwise fashion.

5. The catheter (1, 1D, 1E) according to claim 4, **characterized in that** the element wire constituting the extension portion (222, 222A, 222B1, 222B2, 222C, 222D, 222E) is diameter-thinned toward the distal side thereof.

6. The catheter (1, 1D, 1E) according to any one of claims 1 to 5, **characterized in that** the extension portion (222, 222A, 222B1, 222B2, 222C, 222D, 222E) is positioned between the center axis of the guide wire insertion portion and the center axis of the catheter main body (2).

7. The catheter (1, 1D, 1E) according to any one of claims 1 to 6, **characterized in that** the reinforcement portion (22, 22A, 22B, 22C, 22D, 22E) is composed of a metal material.

## Patentansprüche

1. Katheter (1, 1D, 1E), **dadurch gekennzeichnet, dass** er umfasst:
einen Katheterhauptkörper (2), der eine längliche Form ausbildet und auch ein Lumen (21) aufweist, das sich entlang der Längsrichtung erstreckt;
einen Führungsdrahteinführungsabschnitt (3), der an der distalen Seite des Katheterhauptkörpers (2) angeordnet ist und eine röhrenförmige Form bildet, durch die ein Führungsdraht eingeführt werden kann und die einen distalen Öffnungsabschnitt (37) und einen proximalen Öffnungsabschnitt (36) aufweist; und
einen Verstärkungsabschnitt (22, 22A, 22B, 22C, 22D, 22E), der mit einem Spiralenabschnitt (221, 221A, 221B, 221C, 221D, 221E) versehen ist, in welchem mindestens ein Abschnitt davon an der proximalen Seite entfernt von dem proximalen Öffnungsabschnitt (36) angeordnet ist und unter Verwendung eines Elementdrahtes gewickelt ist,
**dadurch gekennzeichnet, dass** der Verstärkungsabschnitt ferner einen Verlängerungsabschnitt (222, 222A, 222B1, 222B2, 222C, 222D, 222E) aufweist, der durch die Ausdehnung von dem Elementdraht des Spiralenabschnitts (221, 221A, 221B, 221C, 221D, 221E) zu der distalen Seite entfernt von dem proximalen Öffnungsabschnitt (36) gebildet wird, wobei
der Verstärkungsabschnitt (22, 22A, 22B, 22C, 22D, 22E) zwischen der Innenfläche und der Außenfläche einer Rohrwand (23) positioniert ist, welche das Lumen (21) des Katheterhauptkörpers (2) definieren.

2. Katheter (1, 1D, 1E) nach Anspruch 1, **dadurch gekennzeichnet, dass** der distale Verlängerungsabschnitt (225, 225A, 225C), welcher der Endabschnitt an der distalen Seite des Verlängerungsabschnitts (222, 222A, 222B1, 222B2, 222C, 222D, 222E) ist, durch einen gekrümmten Abschnitt gebildet ist, der durch Biegen des Elementdrahtes gebildet ist.

3. Katheter (1, 1D, 1E) nach Anspruch 2, **dadurch gekennzeichnet, dass** in Bezug auf den Verstärkungsabschnitt (22, 22A, 22B, 22C, 22D, 22E) ein durch Biegen eines Stranges des Elementdrahtes gebildeter Biegeabschnitt als distaler Verlängerungsabschnitt (225, 225A, 225C) ausgebildet ist und der Spiralenabschnitt (221, 221A, 221B, 221C, 221D, 221E) durch Wickeln der gegenüberliegenden Seite des Biegeabschnitts des Elementdrahtes in einer Spiralform ausgebildet ist.

4. Katheter (1, 1D, 1E) nach einem der Ansprüche 1 bis 3, **dadurch gekennzeichnet, dass** in Bezug auf den Verlängerungsabschnitt (222, 222A, 222B1, 222B2, 222C, 222D, 222E) die Biegesteifigkeit davon von der proximalen Seite in Richtung der distalen Seite in einer geneigten Weise oder stufenweise abnimmt.

5. Katheter (1, 1D, 1E) nach Anspruch 4, **dadurch gekennzeichnet, dass** der Elementdraht, welcher den Verlängerungsabschnitt (222, 222A, 222B1, 222B2, 222C, 222D, 222E) bildet, in Richtung seiner distalen Seite hin im Durchmesser dünner wird.

6. Katheter (1, 1D, 1E) nach einem der Ansprüche 1 bis 5, **dadurch gekennzeichnet, dass** der Verlängerungsabschnitt (222, 222A, 222B1, 222B2, 222C, 222D, 222E) zwischen der Mittelachse des Führungsdrahteinführungsabschnitts und der Mittelachse des Katheterhauptkörpers (2) positioniert ist.

7. Katheter (1, 1D, 1E) nach einem der Ansprüche 1 bis 6, **dadurch gekennzeichnet, dass** der Verstärkungsabschnitt (22, 22A, 22B, 22C, 22D, 22E) aus einem Metallmaterial besteht.

## Revendications

1. Cathéter (1, 1D, 1E) **caractérisé en ce qu'**il comprend :
un corps principal de cathéter (2) possède une forme allongée et qui comporte également une lumière (21) qui s'étend dans la direction longitudinale ;
une partie d'insertion de fil-guide (3) qui est placée sur le côté distal du corps principal de cathéter (2) et qui forme une forme tubulaire à travers laquelle un fil-guide peut être inséré, et qui comporte une partie d'ouverture distale (37) et une partie d'ouverture proximale (36) ; et
une partie de renforcement (22, 22A, 22B, 22C, 22D, 22E) munie d'une partie de bobine (221, 221A, 221B, 221C, 221D, 221E) dans laquelle au moins une partie de celle-ci est disposée sur le côté proximal opposé à la partie d'ouverture proximale (36) et est enroulée au moyen d'un fil d'élément, **caractérisé en ce que** ladite partie de renforcement comprend en outre une partie de prolongement (222, 222A, 222B1, 222B2, 222C, 222D, 222E) formée en étant étendue depuis le fil d'élément de la partie de bobine (221, 221A, 221B, 221C, 221D, 221E) jusqu'au côté distal opposé à la partie d'ouverture proximale (36), où la partie de renforcement (22, 22A, 22B, 22C, 22D, 22E) est placée entre la surface intérieure et la surface extérieure d'une paroi tubulaire (23) définissant la lumière (21) du corps principal de cathéter (2).

2. Cathéter (1, 1D, 1E) selon la revendication 1, **caractérisé en ce que** la partie distale de prolongement (225, 225A, 225C) qui est la partie d'extrémité sur le côté distal de la partie de prolongement (222, 222A, 222B1, 222B2, 222C, 222D, 222E) est formée par une partie courbe qui est formée en courbant le fil d'élément.

3. Cathéter (1, 1D, 1E) selon la revendication 2, **caractérisé en ce que** concernant la partie de renforcement (22, 22A, 22B, 22C, 22D, 22E), une partie de flexion formée en pliant une ligne du fil d'élément est faite pour constituer la partie distale de prolongement (225, 225A, 225C), et la partie de bobine (221, 221A, 221B, 221C, 221D, 221E) est formée en enroulant le côté opposé de la partie de flexion du fil d'élément en une forme de spirale.

4. Cathéter (1, 1D, 1E) selon l'une quelconque des revendications 1 à 3, **caractérisé en ce que** concernant la partie de prolongement (222, 222A, 222B1, 222B2, 222C, 222D, 222E), la rigidité en flexion de celle-ci diminue en allant du côté proximal vers le côté distal de façon inclinée ou par paliers.

5. Cathéter (1, 1D, 1E) selon la revendication 4, **caractérisé en ce que** le fil d'élément constituant la partie de prolongement (222, 222A, 222B1, 222B2, 222C, 222D, 222E) a un diamètre qui diminue en direction de son côté distal.

6. Cathéter (1, 1D, 1E) selon l'une quelconque des revendications 1 à 5, **caractérisé en ce que** la partie de prolongement (222, 222A, 222B1, 222B2, 222C, 222D, 222E) est placée entre l'axe central de la partie d'insertion de fil-guide et l'axe central du corps principal de cathéter (2).

7. Cathéter (1, 1D, 1E) selon l'une quelconque des revendications 1 à 6, **caractérisé en ce que** la partie de renforcement (22, 22A, 22B, 22C, 22D, 22E) est en métal.
